# EUROPÄISCHE PATENTSCHRIFT

(11) **EP 3 764 127 B1**
(45) Veröffentlichungstag und Bekanntmachung des Hinweises auf die Patenterteilung: **28.08.2024**
(21) Anmeldenummer: 19184975.1
(22) Anmeldetag: 08.07.2019
(51) Int. Cl.: G01T 1/24, H01L 31/115

(54) **RÖNTGENDETEKTOR UND RÖNTGENGERÄT MIT RÖNTGENDETEKTOR**
X-RAY DETECTOR AND X-RAY APPARATUS WITH X-RAY DETECTOR
DÉTECTEUR DE RAYONS X ET APPAREIL À RAYONS X POURVU DE DÉTECTEUR DE RAYONS X

(43) Veröffentlichungstag der Anmeldung: 13.01.2021
(73) Patentinhaber: Siemens Healthineers AG, 91301 Forchheim (DE)
(72) Erfinder: ERGLER, Thorsten, 91054 Erlangen (DE); HOSEMANN, Michael, 91056 Erlangen (DE); WREGE, Jan, 91056 Erlangen (DE)
(74) Vertreter: Siemens Healthineers Patent Attorneys

(56) Entgegenhaltungen:
- DE-A1- 102004 057 533
- DE-A1- 102014 204 042
- JP-A- 2009 018 109

## Beschreibung

Die Erfindung betrifft einen Röntgendetektor aufweisend eine Sensorschicht und einen nachgeschalteten Auslesechip. Darüber hinaus betrifft die Erfindung ein Röntgengerät mit einem solchen Röntgendetektor.

In bilderzeugenden Röntgengeräten werden zunehmend sogenannte direkt konvertierende Röntgendetektoren eingesetzt, wie sie zum Beispiel in der DE 10 2012 202 200 B3 beschrieben sind. Diese Röntgendetektoren sind typischerweise als sogenannte Photonenzählende Röntgendetektoren ausgebildet und weisen als wesentliche Komponenten eine Sensorschicht und einen Auslesechip auf.

Hierbei ist aktuell der Auslesechip direkt an die Sensorschicht angekoppelt, so dass die sehr kleinen analogen Signal-Pulse, welche durch Röntgenquanten im Sensormaterial der Sensorschicht generiert werden, möglichst unverfälscht und rauscharm in den Auslesechip gelangen und dort verarbeitet oder vermessen werden können. Dabei ist eingangsseitig im Auslesechip eine Verstärkerstufe geschaltet, die diese Signal-Pulse verstärkt und so für die weitere Verarbeitung im Auslesechip robust gegen Störeinflüsse macht.

Um nun bei einem solchen Röntgendetektor eine große Sensorfläche zu realisieren, werden typischerweise mehrere Untereinheiten, sogenannte Sensorboards, eng aneinander gekachelt. Für die Bildgebung ist es dabei essenziell, dass der Aufbau des Röntgendetektor oder vielmehr der Sensorfläche möglichst spaltfrei erfolgt. Um dies zu ermöglichen werden üblicherweise Durchkontaktierungen, sogenannte TSVs, in den Auslesechip eingebracht, da dann die Untereinheiten problemlos auch vierseitig direkt aneinandergereiht werden können. Die Herstellung von Auslesechips mit Durchkontaktierungen ist jedoch sehr aufwändig und infolgedessen auch kostenintensiv. Wählt man dagegen andere Ansätze zur Kontaktierung des Auslesechips, bei denen der Auslesechip nicht direkt an die Sensorschicht angekoppelt ist, besteht das Problem, dass längere Leitungen oder gar unterschiedliche Leitungslängen für verschiedene Pixel das Antwortverhalten des Auslesechips stark beeinflussen. Dies kann so weit gehen, dass die Bildqualität problematisch beeinflusst wird.

Die Druckschrift JP 2009 018109 A offenbart für ein Bildgebungsgerät einen Verstärkerschaltkreis mit einem anschließenden Bildsignalauswerteschaltkreis, welcher an einer Unterseite eines Substrats angeordnet ist.

Die Druckschrift DE 10 2004 057533 A1 offenbart einen Detektorriegel bzw. einen Detektor aufweisend einen Modulträger zur mechanischen Halterung von Einzelmodulen jeweils umfassend ein Array von Detektorelementen, welche auf einem Substrat angeordnet sind und eine von diesem baulich getrennte Leiterplatine zur elektrischen Kontaktierung der Einzelmodule. Dabei wird außerdem offenbart, dass auf der von der Röntgenstrahlung abgewandten Seite des Substrats elektronische Bauelemente zur Spannungsaufbereitung, zur Signalverstärkung und zur Entkopplung von Störsignalen mittels Kapazitäten vorgesehen sein können, wobei diese zumindest zum Teil auch auf der Leiterplatine oder einer dem Detektorgestell zugeordneten Elektronikvorrichtung angeordnet sein können.

Ausgehend hiervon liegt der Erfindung die Aufgabe zugrunde, einen vorteilhaft ausgestalteten Röntgendetektor sowie ein vorteilhaft ausgestaltetes Röntgengerät mit einem entsprechenden Röntgendetektor anzugeben.

Diese Aufgabe wird erfindungsgemäß gelöst durch einen Röntgendetektor mit den Merkmalen des Anspruchs 1 sowie durch ein Röntgengerät mit den Merkmalen des Anspruchs 14. Die rückbezogenen Ansprüche beinhalten teilweise vorteilhafte und teilweise für sich selbst erfinderische Weiterbildungen dieser Erfindung. Die im Hinblick auf den Röntgendetektor angeführten Vorteile und bevorzugten Ausgestaltungen sind sinngemäß auch auf das Röntgengerät übertragbar und umgekehrt.

Der erfindungsgemäße Röntgendetektor ist dabei zweckdienlicherweise ausgebildet für eine Nutzung in einem Röntgengerät, beispielsweise einem Computertomographen, und weist eine Sensorschicht sowie einen nachgeschalteten Auslesechip auf, wobei der Auslesechip bevorzugt als sogenannter ASIC-Auslesechip (ASIC:application-specific integrated circuit) ausgestaltet ist.

Hierbei ist der Röntgendetektor und/oder die Sensorschicht typischerweise aus mehreren Untereinheiten oder Modulen aufgebaut, die üblicherweise im Wesentlichen identisch ausgestaltet sind. Ist der Röntgendetektor aus Modulen aufgebaut, so weist üblicherweise jedes Röntgendetektor-Modul eine entsprechende Sensorschicht und zumindest einen entsprechenden nachgeschalteten Auslesechip auf.

Unabhängig davon, ob nun ein modularer Aufbau vorliegt oder nicht, ist erfindungsgemäß zwischen der Sensorschicht und dem Auslesechip eine erste Verstärkerstufe zwischengeschaltet, wobei diese erste Verstärkerstufe erfindungsgemäß ebenfalls als eine Art Schicht, also eine Art funktionelle Schicht, ausgebildet ist. Die erste Verstärkerstufe ist dabei typischerweise direkt oder unmittelbar an der Sensorschicht platziert und erfindungsgemäß direkt oder über Lötverbindungen mit dieser elektrisch leitfähig verbunden.

Dabei ist die erste Verstärkerstufe bevorzugt über sehr kurze und insbesondere gleichartige elektrisch leitfähige Verbindungen oder Überbrückungen, also insbesondere Lötverbindungen, mit der Sensorschicht verbunden, sodass diese elektrisch leitfähigen Verbindungen keine signifikanten parasitären Kapazitäten und/oder Widerstände aufweisen. Durch die erste Verstärkerstufe werden dann im Betrieb des Röntgendetektors die Sensorsignale oder Sensor-Pulse aus der Sensorschicht durch die erste Verstärkerstufe verstärkt, noch bevor parasitäre Kapazitäten und/oder Widerstände wirksam werden und in der Folge sind dann die verstärkten Sensorsignale unempfindlicher gegen Effekte von parasitären Kapazitäten und/oder Widerständen in der elektrisch leitfähige Verbindung hin zum Auslesechip.

Somit werden dann die verstärkten Sensorsignale nahezu unbeeinträchtigt von den weiteren Leitungslängen und/oder den weiteren Leitungskapazitäten an den Auslesechip herangeführt und dort weiterverarbeitet. Hierdurch ergeben sich neue Möglichkeiten hinsichtlich der Ausgestaltung der elektrisch leitfähigen Verbindungen hin zum Auslesechip. Dabei wird es insbesondere ermöglicht, unabhängiger von den Eingangskapazitäten hin zu den Eingängen des Auslesechips zu werden.

Die Grundidee kann hierbei auch darin gesehen werden, dass ausgehend von einem Röntgendetektor mit einem Auslesechip nach dem Stand der Technik eine Verlagerung der Verstärkerfunktion oder zumindest eines Teils der Verstärkerfunktion des Auslesechips aus dem Auslesechip heraus in eine weitere oder separate Komponentenebene vorgenommen wird. Diese neue, separate Komponentenebene wird dabei nahe der Sensorschicht platziert. Dies ermöglicht eine weite räumliche Trennung von Sensorschicht und Auslesechip unter Berücksichtigung der Problematik, dass die Sensorschicht sehr kleine analoge Sensorsignale im pA-Bereich liefert, aber dennoch eine genaue Detektion/Auswertung der Sensorsignale im Auslesechip erfolgen soll.

Weiter ist der Röntgendetektor als sogenannter direkt konvertierender Röntgendetektor oder Photonenzählender Röntgendetektor ausgebildet und dementsprechend ist beispielsweise CdTe, CdZnTe, CdZnTeSe, CdTeSe, CdNnTe, InP, TIBr₂, oder HgI₂ Hauptbestandteil der Sensorschicht. Die Sensorschicht oder Konverterschicht dient dabei nach an sich bekanntem Prinzip zur direkten Konvertierung von Röntgenstrahlung in elektrische Signale, also Sensorsignale, wobei die Sensorsignale typischerweise als Strom-Pulse vorliegen.

Außerdem weist der Röntgendetektor oder ein jedes Röntgendetektor-Modul erfindungsgemäß eine Vielzahl von Pixeln auf, wobei zu jedem Pixel ein Volumenelement der Sensorschicht gehört sowie eine daran elektrisch leitfähig angebundene Signalverarbeitungs-Einheit. Teil einer solchen Signalverarbeitungs-Einheit ist dabei in der Regel eine Verstärkerschaltung der ersten Verstärkerstufe und eine Ausleseschaltung im Auslesechip. Hierbei werden die in der Sensorschicht generierten Sensorsignale der verschiedenen Pixel typischerweise zumindest anfangs unabhängig voneinander verstärkt, verarbeitet und ausgewertet und dementsprechend weist dann die erste Verstärkerstufe erfindungsgemäß für jedes Pixel eine eigene Verstärkerschaltung auf und der Auslesechip weist typischerweise einen Signaleingang pro Pixel auf. Es findet also eine pixelweise Verstärkung durch die erste Verstärkerstufe statt.

Die Verstärkerschaltungen der ersten Verstärkerstufe sind hierbei typischerweise mittels einfacher TransistorSchaltungen realisiert, die in den meisten Fällen durch Nutzung einer CMOS-Technologie hergestellt sind. Davon unabhängig wird je nach Anwendungsfall mittels einer entsprechenden Verstärkerschaltung eine Stromverstärkung mit einem Verstärkungsfaktor realisiert, dessen Wert im Bereich von etwa 2 bis etwa 100 und insbesondere im Bereich von etwa 2 bis etwa 50 liegt. Alternativ oder ergänzend hierzu wird in der ersten Verstärkerstufe eine Strom-zu-Spannungswandlung realisiert, wobei in diesem Fall die Stromwerte vorzugsweise mit einem Faktor im Bereich von etwa 1 mV/nA bis etwa 100 mV/nA und insbesondere im Bereich von etwa 1 mV/nA bis etwa 50 mV/nA in Spannungswerte umgewandelt werden.

In jedem Fall aber wird mittels der ersten Verstärkerstufe bevorzugt eine aktive Verstärkung vorgenommen und dementsprechend wird die erste Verstärkerstufe im Betrieb mit elektrischer Leistung versorgt. D. h., dass die erste Verstärkerstufe einen Versorgungseingang für elektrische Leistung aufweist, über die im Betrieb der ersten Verstärkerstufe elektrische Leistung zur aktiven Verstärkung von Sensorsignalen zugeführt wird.

Erfindungsgemäß weist der Auslesechip zudem eine zweite Verstärkerstufe auf, die in den Auslesechip integriert ist und somit Teil des Auslesechips ist. Die zweite Verstärkerstufe weist dabei eine Verstärkerschaltung pro Pixel auf, wobei eine jede solche Verstärkerschaltung typischerweise einem Eingang unmittelbar nachgeschaltet ist. Die Verstärkerschaltungen der zweiten Verstärkerstufe sind hierbei vorzugsweise einfacher gehalten als bei einem Auslesechip nach dem Stand der Technik, da ja bereits durch die erste Verstärkerstufe im Betrieb eine Verstärkung erfolgt.

Die mit einer Verstärkerschaltung der zweiten Verstärkerstufe realisierte Verstärkung richtet sich dabei üblicherweise nach der durch die erste Verstärkerstufe realisierten Verstärkung und/oder danach, ob mittels der ersten Verstärkerstufe eine Strom-zu-Spannungswandlung realisiert ist oder wird. Hierbei ist die zweite Verstärkerstufe bevorzugt derart ausgestaltet, dass spätestens an den Ausgängen der zweiten Verstärkerstufe Spannungssignale vorliegen und zwar insbesondere Spannungssignale, deren typische Pulshöhen je nach Energie der Röntgenquanten im Bereich von etwa 10 mV bis etwa 150 mV liegen. Je nach Auslegung, also beispielsweise je nach gewählten Herstellungsprozess und dessen bevorzugter Betriebsspannung sowie der geforderten Linearität (Vermeidung von Sättigung durch sogenanntes Pile-Up), schwanken diese Werte gegebenenfalls noch um einen Faktor bis etwa 5. Der typischerweise sehr große Schwankungsbereich wird dabei häufig durch viele unterschiedliche Faktoren beeinflusst.

Da sich also die mit einer Verstärkerschaltung der zweiten Verstärkerstufe realisierte Verstärkung vorzugsweise nach der Ausgestaltung der ersten Verstärkerstufe richtet, wird in einigen Fällen sogar auf eine größere oder gar auf eine gezielte Verstärkung mittels der zweiten Verstärkerstufe verzichtet. In einem solchen Fall erfolgt durch eine Verstärkerschaltung der zweiten Verstärkerstufe vor allem eine Filterung des mittels der ersten Verstärkerstufe verstärkten Signals, welche auch als Pulsformung bezeichnet wird, und/oder eine Strom-zu-Spannungswandlung. Typische Verstärkungsfaktoren liegen dann lediglich im Bereich von etwa 1 bis etwa 10.

Weiter weist der Auslesechip üblicherweise eine Filterschaltung pro Pixel auf, die typischerweise der Verstärkerschaltung der zweiten Verstärkerstufe nachgeschaltet ist. Außerdem weist der Auslesechip üblicherweise eine Zählerschaltung pro Pixel auf, die dann typischerweise die pixelweise Verarbeitung der Sensorsignale abschließt.

Von Vorteil ist des Weiteren eine Ausführung des Röntgendetektors, bei der zwischen der Sensorschicht und dem Auslesechip ein sogenannter Interposer zwischengeschaltet ist. Der Interposer, auch Umverdrahtungseinheit oder Umkontaktierungseinheit genannt, dient dabei dazu, zwei Einheiten elektrisch leitfähig miteinander zu verbinden, die jeweils mehrere elektrische Kontakte oder Anschlüsse aufweisen, bei denen die elektrischen Kontakte oder Anschlüsse jedoch unterschiedlich angeordnet sind, sodass einfache direkte Verbindungen, beispielsweise Lötverbindungen, nicht ausgebildet werden können.

Ist ein solcher Interposer vorgesehen, so ist dieser weiter bevorzugt der ersten Verstärkerstufe nachgeschaltet und dementsprechend ist dann die erste Verstärkerstufe bevorzugt zwischen Sensorschicht und Interposer zwischengeschaltet.

Gemäß einer Ausführungsvariante ist die erste Verstärkerstufe weiter mittels eines Verfahrens auf Basis einer TFT-Technologie (TFT: thin-film transistor) gefertigt. Diese Technologie ermöglicht es, Transistoren auf Glas zu fertigen, und wird bereits in Großserie eingesetzt, zum Beispiel zur Fertigung von Displays.

Insbesondere wenn die erste Verstärkerstufe auf Basis einer TFT-Technologie gefertigt ist, ist es außerdem von Vorteil, wenn der Interposer als ein Glas-Interposer ausgestaltet ist, wobei dann weiter bevorzugt die erste Verstärkerstufe auf dem Glas-Interposer mittels eines Verfahrens auf Basis einer TFT-Technologie gefertigt ist. Dabei sind Technologien zur Fertigung von Durchkontaktierungen und/oder Umverdrahtungsebenen in Glas bereits verfügbar und reine Interposer aus Glas (ohne Verstärkerstufe) sind bereits bekannt. Diese Technologien werden nun im Falle einer Ausführungsvariante des Röntgendetektors genutzt, um einen Interposer aus Glas zu fertigen, der zusätzlich die erste Verstärkerstufe aufweist.

Alternativ wird anstatt einem Interposer aus Glas ein Interposer auf der Basis eines Halbleitermaterials, beispielsweise Silizium, genutzt und in diesem Fall weist dann der Röntgendetektor einen solchen Interposer auf der Basis eines Halbleitermaterials auf. Ein derartige Interposer ist dann typischerweise aus mehreren Schichten oder Lagen aufgebaut und bevorzugt ist die erste Verstärkerstufe durch zumindest eine dieser Lagen ausgebildet.

In vorteilhafter Weiterbildung ist der die erste Verstärkerstufe ausbildenden Lage des Interposers eine Umverteilungs-Lage (RDL: Re-Distribution-Layer) nachgeschaltet und üblicherweise sind alle Umverteilungs-Lagen des Interposers der die erste Verstärkerstufe ausbildenden Lage nachgeschaltet.

Der Interposer besteht dann also beispielsweise aus mehreren Lagen, wobei eine Lage aus Silizium als Basismaterial ausgebildet ist und wobei in diese Lage die erste Verstärkerstufe integriert ist. Hierzu lässt sich zum Beispiel eine CMOS-Technologie nutzen, mittels der Verstärkerschaltungen einzeln oder in Gruppen von mehreren Verstärkern in Lagen des Interposers integriert werden. Diese Lagen haben hierzu typischerweise Aussparungen. Zudem weist ein solcher Interposer üblicherweise zumindest eine Umverteilungs-Lage auf, in der zum Beispiel Leiterbahnen aus Kupfer verlaufen.

Des Weiteren ist eine Ausführungsvariante des Röntgendetektors von Vorteil, bei der die erste Verstärkerstufe auf einer Folie realisiert ist. Hierbei ist die erste Verstärkerstufe bevorzugt auf die Folie aufgedruckt und dementsprechend sind dann die elektrischen und/oder elektronischen Bausteine der Verstärkerstufe durch eine Drucktechnik auf die Folie aufgebracht.

Eine solche Folie ist dann zweckdienlicherweise auf die Sensorschicht oder, sofern vorhanden, auf einen Interposer auflaminiert, also zum Beispiel aufgeklebt. Bevorzugt erfolgt die Befestigung mittels einer Art Klebung, wobei für elektrische Kontakte vorzugsweise ein Leitkleber genutzt wird.

Als vorteilhaft wird weiter eine Ausführungsvariante des Röntgendetektors angesehen, bei der die erste Verstärkerstufe an der Sensorschicht angebracht ist. Die erste Verstärkerstufe ist in diesem Fall bevorzugt durch eine Halbleiterschicht oder Halbleiterlage realisiert, die insbesondere durch Epilepsie auf die Sensorschicht aufgebracht ist, also entweder direkt oder indirekt über eine Zwischenlage oder Zwischenschicht.

Es wird also zum Beispiel eine Siliziumschicht oder Siliziumlage durch Epitaxie auf der Sensorschicht abgeschieden und in diese Siliziumschicht wird dann die erste Verstärkerstufe implantiert. Hierbei wird je nach Anwendungsfall gegebenenfalls zunächst eine Zwischenlage aus Siliziumdioxid auf der Sensorschicht abgeschieden und nachfolgend wird dann die Siliziumschicht ausgebildet.

Zweckdienlich ist zudem eine Ausführungsvariante des Röntgendetektors, bei der zwischen der ersten Verstärkerstufe und dem Auslesechip eine Leiterplatte zwischengeschaltet ist. Die Leiterplatte ist dabei je nach Ausführungsvariante als starre Leiterplatte, als flexible Leiterplatte oder als teils starre und teils flexible Leiterplatte ausgebildet.

Ausführungsbeispiele der Erfindung werden nachfolgend anhand einer schematischen Zeichnung näher erläutert. Darin zeigen:
- FIG 1: in einer ausschnittsweisen Schnittdarstellung eine erste Ausführung eines Röntgendetektors,
- FIG 2: in einer semitransparenten Ansicht ein Röntgengerät mit dem Röntgendetektor,
- FIG 3: in einem Schaltbild ein Pixel des Röntgendetektors,
- FIG 4: in einer ausschnittsweisen Schnittdarstellung eine zweite Ausführung des Röntgendetektors,
- FIG 5: in einer ausschnittsweisen Schnittdarstellung eine dritte Ausführung des Röntgendetektors und
- FIG 6: in einer ausschnittsweisen Schnittdarstellung eine vierte Ausführung des Röntgendetektors.

Einander entsprechende Teile sind in allen Figuren jeweils mit den gleichen Bezugszeichen versehen.

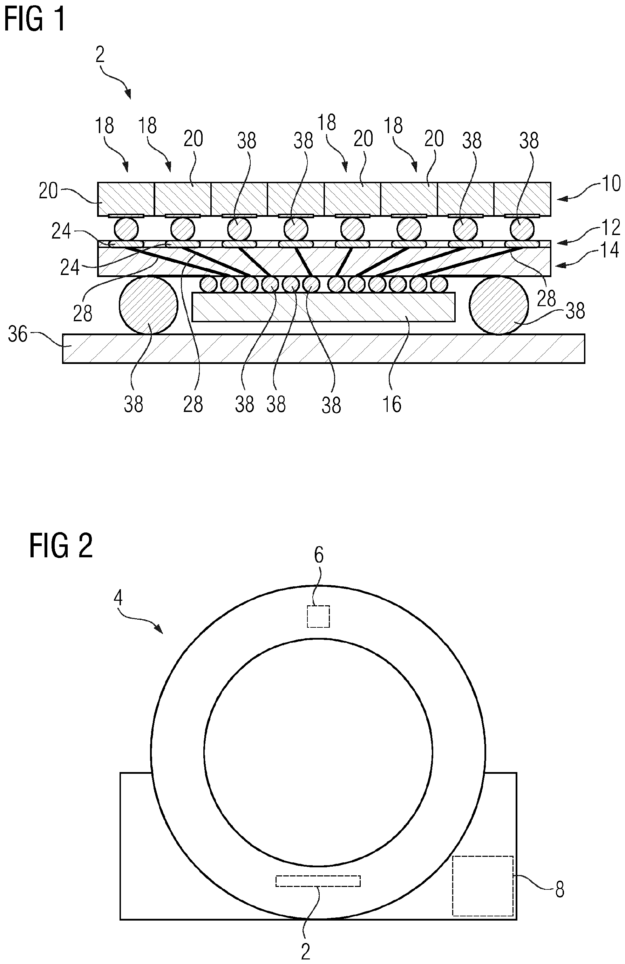

Ein nachfolgend beschriebener Röntgendetektor 2 ist als direkt konvertierender Röntgendetektor 2 ausgebildet und in FIG 1 in einer ersten Ausführungsvariante schematisch dargestellt.

Im Ausführungsbeispiel ist der Röntgendetektor 2 dabei Bestandteil eines Röntgengeräts 4, welches exemplarisch als Computertomograph ausgebildet und in FIG 2 skizziert ist. Im Röntgengerät 4 ist der Röntgendetektor 2 ergänzt durch eine Röntgenstrahlungsquelle 6. Zudem weist das Röntgengerät 4 eine Steuereinheit 8 auf.

Der Röntgendetektor 2 ist, wie bereits dargelegt, als direkt konvertierender Röntgendetektor 2 ausgestaltet und weist als wesentliche Komponenten eine Konverterschicht oder Sensorschicht 10, eine erste Verstärkerstufe 12, einen Interposer 14 sowie einen ASIC-Auslesechip 16 auf.

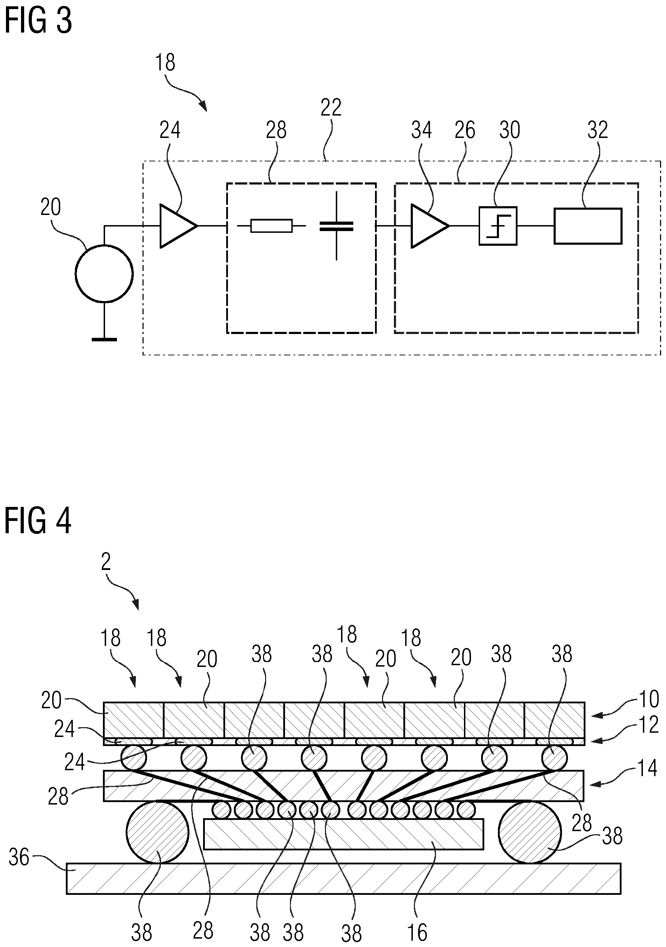

Außerdem weist der Röntgendetektor 2 eine Vielzahl an Pixeln 18 auf oder bildet eine Vielzahl an Pixeln 18 aus, wobei zu jedem Pixel 18 ein Volumenelement 20 der Sensorschicht 10 gehört sowie eine Signalverarbeitungs-Einheit 22. In FIG 3 ist exemplarisch ein solches Pixel 18 in Form eines vereinfachten Blockschaltbildes wiedergegeben.

Eine jede Signalverarbeitungs-Einheit 22 wiederum wird im Ausführungsbeispiel gebildet durch eine erste Verstärkerschaltung 24, die Teil der ersten Verstärkerstufe 12 ist, sowie eine Ausleseschaltung 26, die Teil des ASIC-Auslesechips 16 ist, wobei die erste Verstärkerschaltung 24 über eine Interposer-Verbindung 28, die Teil des Interposers 14 ist, mit der Ausleseschaltung 26 verbunden ist. Somit weist also jedes Pixel 18 ein Volumenelement 20 sowie eine Signalverarbeitungs-Einheit 22 mit einer ersten Verstärkerschaltung 24, mit einer Interposer-Verbindung 28 sowie mit einer Ausleseschaltung 26 auf. Hierbei weist eine solche Interposer-Verbindung 28 typischerweise eine charakteristische parasitäre Kapazität sowie einen charakteristischen parasitären Widerstand auf.

Infolge dieser Ausgestaltung wird dann im Betrieb des Röntgendetektors 2 ein in einem Volumenelement 20 eines Pixels 18 generiertes Sensorsignal zunächst in der an das Volumenelement 20 angebundenen ersten Verstärkerschaltung 24 des entsprechenden Pixels 18 verstärkt. Nachfolgend wird das verstärkte Sensorsignal über die Interposer-Verbindung 28 dieses Pixels 18 hin zum ASIC-Auslesechips 16 weitergeleitet und dort in der Ausleseschaltung 26 des Pixels 18 weiterverarbeitet.

Im Ausführungsbeispiel arbeiten die Signalverarbeitungs-Einheiten 22 der Pixel 18 unabhängig voneinander und in jedem Pixel 18 werden die auftreffenden Röntgenquanten gezählt, wobei die Ausleseschaltung 26 im ASIC-Auslesechip 16 hierfür typischerweise einen Komparator 30 und eine Zähleinheit 32 aufweist. Zudem weist im Ausführungsbeispiel jede Ausleseschaltung 26 eine zweite Verstärkerschaltung 34 auf und alle zweiten Verstärkerschaltungen 34 des ASIC-Auslesechips 16 bilden zusammen eine zweite Verstärkerstufe aus.

In FIG 1 ist nun eine erste Ausführungsvariante des Röntgendetektors 2 in einer ausschnittsweisen Schnittdarstellung schematisch wiedergegeben. Aus der Darstellung ist zu entnehmen, dass der Interposer 14 zwischen Sensorschicht 10 und ASIC-Auslesechip 16 zwischengeschaltet ist und dass der ASIC-Auslesechip 16 schließlich elektrisch leitfähig mit einer Leiterplatte 36 verbunden oder an die Leiterplatte 36 angebunden ist.

Weiter ist zwischen Sensorschicht 10 und Interposer 14 die erste Verstärkerstufe 12 zwischengeschaltet, wobei zwischen der Sensorschicht 10 und der ersten Verstärkerstufe 12 im Ausführungsbeispiel einfache Lötverbindungen 38 ausgebildet sind. Dabei ist über eine solche Lötverbindung 38 ein Volumenelement 20 mit einer ersten Verstärkerschaltung 24 der ersten Verstärkerstufe 12 elektrisch leitfähig verbunden.

Die erste Verstärkerstufe 12 ist hierbei je nach Ausgestaltungsvariante beispielsweise auf einer nicht explizit dargestellten Folie realisiert, also zum Beispiel auf eine Folie aufgedruckt, und die Folie ist dann zum Beispiel auf die Sensorschicht 10 oder den Interposer 14 auflaminiert.

Alternativ ist die erste Verstärkerstufe 12 mittels eines Verfahrens auf Basis einer TFT-Technologie gefertigt und auf dem Interposer 14 realisiert, der dann bevorzugt als Glas-Interposer ausgebildet ist.

Eine zweite Ausführungsvariante des Röntgendetektors 2 ist in FIG 4 dargestellt, zumindest in vereinfachter Form. Hier ist die erste Verstärkerstufe 12 direkt an der Sensorschicht 10 ausgebildet, wobei hierzu im Ausführungsbeispiel eine Siliziumschicht oder Siliziumlage durch Epitaxie auf die Sensorschicht 10 aufgebracht ist und wobei die die erste Verstärkerstufe 12 ausbildenden nicht näher dargestellten Halbleiter-Schaltungen in diese Siliziumschicht integriert sind oder in der Siliziumschicht realisiert sind. Silizium stellt dabei nur ein geeignetes Materialbeispiel dar. Insbesondere wenn als Sensormaterial der Sensorschicht 10 Galliumarsenid zum Einsatz kommt, ist es ebenso günstig, wenn eine Epitaxieschicht mit Halbleiter-Schaltungen und/oder Verstärkern in Galliumarsenidtechnologie realisiert wird.

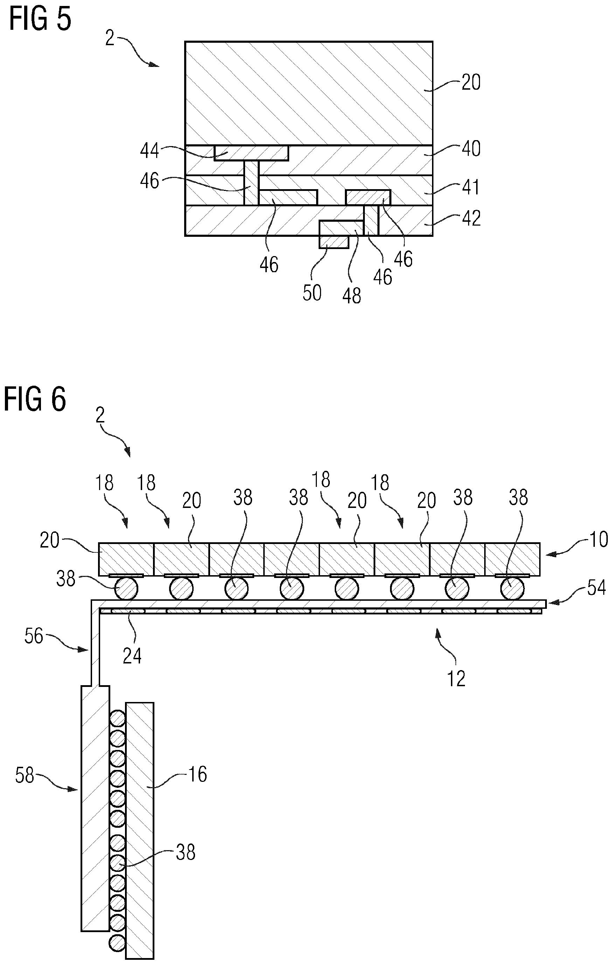

Eine weitere Ausführungsvariante des Röntgendetektors 2 ist in FIG 5 angedeutet. Hier sind mehrere Lagen 40,41,42, nämlich eine erste Lage 40, eine Zwischenlage 41 und eine Umverteilungs-Lage 42, an der Sensorschicht 10 angebracht, wobei in der Zwischenlage 41 die erste Verstärkerstufe 12 realisiert ist. Da die Umkontaktierung im Ausführungsbeispiel gemäß FIG 5 letzten Endes über alle drei gezeigten Lagen 40,41,42 hinweg erfolgt, bilden im Prinzip alle drei Lagen 40,41,42 in diesem Ausführungsbeispiel den Interposer 14 aus, wobei bei diesem Interposer 14 in der Zwischenlage 41 die erste Verstärkerstufe 12 ausgebildet ist.

Gezeigt ist dabei in FIG 5 ein prinzipieller Aufbau im Bereich eines Pixels 18. An das Volumenelement 20 des Pixels schließt sich zunächst ein Metallkontakt 44 an. Jener Metallkontakt 44 ist dabei eingebettet die erste Lage 40 aus Silizium. An diese erste Lage 40 schließen sich zwei weitere Lagen aus Silizium an, wobei in der Zwischenlage 41 durch Dotierung Schaltungs-Strukturen 46 ausgebildet sind, die die erste Verstärkerschaltung 24 dieses Pixels 18 ausbilden. Die dritte Lage 42, die als Umverteilungs-Lage 42 dient, weist Leiterbahnen 48 aus Kupfer 48 auf, durch die ein nach außen geführter Ausgang 50 des Interposers so positioniert ist, dass dieser über eine Lötverbindung 38 direkt mit einem Eingang am ASIC-Auslesechip 16 elektrisch leitfähig verbunden verbindbar ist und im Röntgendetektor 2 auch verbunden ist.

Die dritte Ausführung des Röntgendetektors 2 gemäß FIG 5 ist weiterhin in einigen Fällen dahingehend angepasst, dass die Zwischenlage 41 durch mehrere vergleichbare Zwischenlagen/Zwischenschichten ersetzt ist. Grund hierfür ist dann typischerweise, dass für die erste Verstärkerschaltung 24 auch noch zusätzliche Verdrahtungen beispielsweise zur Stromversorgung und/oder zur Verteilung der Signale realisiert sind.

Solche Verdrahtungen sind dann häufig in mehreren Schichten realisiert.

Ein viertes Ausführungsbeispiel des Röntgendetektors 2 ist in FIG 6 schematisch wiedergegeben. Hier ist der ASIC-Auslesechip 16 über eine teils flexible teils starre Leiterplatte 52 an die erste Verstärkerstufe 12 angebunden, wobei diese Leiterplatte 52 als eine Art Interposer 14 fungiert. Die erste Verstärkerstufe 12 selbst ist dabei über nicht näher dargestellte Durchkontaktierungen sowie Lötverbindungen 38 mit der Sensorschicht 10 elektrisch leitfähig verbunden. Außerdem ist die erste Verstärkerstufe 12 auf einem dünnen, starren ersten Teil 54 der Leiterplatte 52 realisiert. Sie ist in einem einfachen und preisgünstigen Schaltkreisprozess gefertigt. Jener erste Teil 54 der Leiterplatte 52 ist über einen flexiblen, zweiten Teil 56 mit einem wiederum starren dritten Teil 58 verbunden, an dem der ASIC-Auslesechip 16 angebunden ist. Der ASIC-Auslesechip 16 ist in einem aufwändigeren und teureren Prozess gefertigt und auf einer wesentlich kleineren Fläche realisiert.

Die Erfindung ist nicht auf das vorstehend beschriebene Ausführungsbeispiel beschränkt. Vielmehr können auch andere Varianten der Erfindung von dem Fachmann hieraus abgeleitet werden, ohne den Gegenstand der Erfindung zu verlassen, der nur durch die Ansprüche definiert wird.

## Patentansprüche

1. Röntgendetektor (2) mit einer Vielzahl von Pixeln und aufweisend eine Sensorschicht (10) zur direkten Konvertierung von Röntgenstrahlung, wobei zu jedem Pixel ein Volumenelement der Sensorschicht (10) gehört, und einen nachgeschalteten Auslesechip (16) wobei zwischen Sensorschicht (10) und Auslesechip (16) eine erste Verstärkerstufe (12) zwischengeschaltet ist, wobei die erste Verstärkerstufe als Schicht ausgebildet ist, welche direkt oder über Lötverbindungen mit der Sensorschicht elektrisch leitfähig verbunden ist, aufweisend für jedes Pixel eine eigene Verstärkerschaltung zur pixelweisen Verstärkung, und der Auslesechip (16) eine zweite Verstärkerstufe (34) aufweist, aufweisend eine zweite Verstärkerschaltung pro Pixel.

2. Röntgendetektor (2) nach Anspruch 1, wobei zwischen Sensorschicht (20) und Auslesechip (16) ein Interposer (14) zwischengeschaltet ist.

3. Röntgendetektor (2) nach Anspruch 2, wobei die erste Verstärkerstufe (12) zwischen Sensorschicht (10) und Interposer (14) zwischengeschaltet ist.

4. Röntgendetektor (2) nach einem der Ansprüche 1 bis 3, wobei die erste Verstärkerstufe (12) mittels eines Verfahrens auf Basis einer TFT-Technologie gefertigt ist.

5. Röntgendetektor (2) nach Anspruch 2 oder 3, wobei zwischen Sensorschicht (10) und Auslesechip (16) als Interposer (14) ein Glas-Interposer zwischengeschaltet ist und dass die erste Verstärkerstufe (12) auf dem Glas-Interposer mittels eines Verfahrens auf Basis einer TFT-Technologie gefertigt ist.

6. Röntgendetektor (2) nach Anspruch 2 oder 3, wobei zwischen Sensorschicht (10) und Auslesechip (16) der Interposer (14) zwischengeschaltet ist, dass der Interposer (14) eine Anzahl Lagen (40,41,42) aufweist und dass die erste Verstärkerstufe (12) durch zumindest eine dieser Lagen (41) ausgebildet ist.

7. Röntgendetektor (2) nach Anspruch 6, wobei die die erste Verstärkerstufe (12) ausbildende Lage (41) einer Umverteilungs-Lage (42) vorgeschaltet ist.

8. Röntgendetektor (2) nach einem der Ansprüche 1 bis 3, wobei die erste Verstärkerstufe (12) auf einer Folie realisiert ist.

9. Röntgendetektor (2) nach Anspruch 8, wobei die erste Verstärkerstufe (12) auf die Folie aufgedruckt ist.

10. Röntgendetektor (2) nach Anspruch 8 oder 9, wobei die Folie auf die Sensorschicht (12) oder einen Interposer (14) auflaminiert ist.

11. Röntgendetektor (2) nach einem der Ansprüche 1 bis 3, wobei die erste Verstärkerstufe (12) an der Sensorschicht (10) angebracht ist.

12. Röntgendetektor (2) nach einem der Ansprüche 1 bis 3, wobei die erste Verstärkerstufe (12) mittels einer Halbleiterschicht (41) realisiert ist, die durch Epitaxie auf die Sensorschicht (10) aufgebracht ist.

13. Röntgendetektor (2) nach Anspruch 11 oder 12, wobei zwischen der ersten Verstärkerstufe (12) und dem Auslesechip (16) eine Leiterplatte (52) zwischengeschaltet ist.

14. Röntgengerät (4) aufweisend eine Röntgenstrahlungsquelle (6) sowie einen Röntgendetektor (2) nach einem der vorherigen Ansprüche.

## Claims

1. X-ray detector (2) with a plurality of pixels and having a sensor slice (10) for directly converting x-ray radiation, wherein a volume element of the sensor slice (10) forms part of each pixel, and a downstream read-out chip (16),
wherein a first amplifier stage (12) is interconnected between sensor slice (10) and read-out chip (16), wherein the first amplifier stage is embodied as a slice which is connected to the sensor slice in an electrically conductive manner directly or via solder connections, having a separate amplifier circuit for the amplification pixel by pixel for each pixel, and the readout chip (16) has a second amplifier stage (34), having a second amplifier circuit per pixel.

2. X-ray detector (2) according to claim 1,
wherein an interposer (14) is interconnected between the sensor layer (20) and read-out chip (16).

3. X-ray detector (2) according to claim 2,
wherein the first amplifier stage (12) is interconnected between the sensor slice (10) and interposer (14).

4. X-ray detector (2) according to one of claims 1 to 3, wherein the first amplifier stage (12) is manufactured by means of a method based on TFT technology.

5. X-ray detector (2) according to claim 2 or 3,
wherein a glass interposer is interconnected as interposer (14) between the sensor slice (10) and read-out chip (16) and that the first amplifier stage (12) is manufactured on the glass interposer by means of a method based on TFT technology.

6. X-ray detector (2) according to claim 2 or 3,
wherein the interposer (14) is interconnected between the sensor slice (10) and the read-out chip (16), that the interposer (14) has a number of layers (40, 41, 42) and that the first amplifier stage (12) is embodied by means of at least one of these layers (41).

7. X-ray detector (2) according to claim 6,
wherein the layer (41) forming the first amplifier stage (12) is arranged upstream of a redistribution layer (42).

8. X-ray detector (2) according to one of claims 1 to 3, wherein the first amplifier stage (12) is realized on a film.

9. X-ray detector (2) according to claim 8,
wherein the first amplifier stage (12) is printed on the film.

10. X-ray detector (2) according to claim 8 or 9,
wherein the film is laminated onto the sensor slice (12) or an interposer (14).

11. X-ray detector (2) according to one of claims 1 to 3, wherein the first amplifier stage (12) is attached to the sensor slice (10).

12. X-ray detector (2) according to one of claims 1 to 3, wherein the first amplifier stage (12) is realized by means of a semiconductor slice (41), which is applied to the sensor slice (10) by means of epitaxy.

13. X-ray detector (2) according to claim 11 or 12,
wherein a conductor board (52) is interconnected between the first amplifier stage (12) and the read-out chip (16).

14. X-ray device (4) having an x-ray radiation source (6) and an x-ray detector (2) according to one of the preceding claims.

## Revendications

1. Détecteur (2) de rayons X comprenant une pluralité de pixels et comportant une couche (10) de capteur pour la conversion directe du rayonnement X, dans lequel un élément de volume de la couche (10) de capteur appartient à chaque pixel, et une puce (16) de lecture montée en aval, dans lequel un premier étage (12) amplificateur est monté entre la couche (10) de capteur et la puce (16) de lecture, dans lequel le premier étage amplificateur est constitué sous la forme d'une couche, qui est reliée d'une manière conductrice de l'électricité à la couche de capteur directement ou par des brasures, comportant pour chaque pixel un circuit amplificateur propre pour l'amplification pixel par pixel, et la puce (16) de lecture a un deuxième étage (34) amplificateur, comportant un deuxième circuit amplificateur par pixel.

2. Détecteur (2) de rayons X suivant la revendication 1,
dans lequel un interposeur (14) est monté entre la couche (20) de capteur et la puce (16) de lecteur.

3. Détecteur (2) de rayons X suivant la revendication 2,
dans lequel le premier étage (12) amplificateur est monté entre la couche (10) de capteur et l'interposeur (14).

4. Détecteur (2) de rayons X suivant l'une des revendications 1 à 3,
dans lequel le premier étage (12) amplificateur est fabriqué au moyen d'un procédé à base d'une technologie TFT.

5. Détecteur (2) de rayons X suivant la revendication 2 ou 3, dans lequel un interposeur en verre est monté comme interposeur (14) entre la couche (10) de capteur et la puce (16) de lecture, et en ce que le premier étage (12) amplificateur est fabriqué sur l'interposeur en verre au moyen d'un procédé à base d'une technologie TFT.

6. Détecteur (2) de rayons X suivant la revendication 2 ou 3, dans lequel l'interposeur (14) est monté entre la couche (10) de capteur et la puce (16) de lecture, en ce que l'interposeur (14) a un certain nombre de couches (40, 41, 42) et en ce que le premier étage (12) amplificateur est constitué par au moins l'une de ces couches (41).

7. Détecteur (2) de rayons X suivant la revendication 6,
dans lequel la couche (41) constituant le premier étage (12) amplificateur est monté en amont d'une couche (42) de re-répartition.

8. Détecteur (2) de rayons X suivant l'une des revendications 1 à 3,
dans lequel le premier étage (12) amplificateur est réalisé sur une feuille.

9. Détecteur (2) de rayons X suivant la revendication 8,
dans lequel le premier étage (12) amplificateur est imprimé sur la feuille.

10. Détecteur (2) de rayons X suivant la revendication 8 ou 9, dans lequel la feuille est laminée sur la couche (12) de capteur ou sur un interposeur (14).

11. Détecteur (2) de rayons X suivant l'une des revendications 1 à 3,
dans lequel le premier étage (12) amplificateur est mis sur la couche (10) de capteur.

12. Détecteur (2) de rayons X suivant l'une des revendications 1 à 3,
dans lequel le premier étage (12) amplificateur est réalisé au moyen d'une couche (41) à semiconducteur, qui est déposée par épitaxie sur la couche (10) de capteur.

13. Détecteur (2) de rayons X suivant la revendication 11 ou 12, dans lequel une plaquette (52) à circuit imprimé est montée entre le premier étage (12) amplificateur et la puce (16) de lecture.

14. Appareil (4) à rayons X, comportant une source (6) de rayonnement X ainsi qu'un détecteur (2) de rayons X suivant l'une des revendications précédentes.
